# DEMANDE DE BREVET EUROPEEN

(11) **EP 2 446 820 A1**
(43) Date de publication de la demande: **02.05.2012**
(21) Numéro de dépôt: 10306194.1
(22) Date de dépôt: 29.10.2010
(51) Int. Cl.: A61B 6/00, A61B 6/10

(54) **Dispositif d'imagerie par rayons X avec un bras en C et une unité anti-collisions**

(71) Demandeur: General Electric Company, Schenectady, NY 12345 (US)
(72) Inventeur: Trousset, Yves, 78533, Buc (FR); Bouvier, Bernard, 78533, Buc (FR)
(74) Mandataire: Callon de Lamarck, Jean-Robert

(57) **Abrégé**

L'invention concerne un procédé de contrôle d'un dispositif d'imagerie médicale au moyen d'une unité anti-collision d'un dispositif d'imagerie médicale, le dispositif d'imagerie médicale comprenant; une source (3) de rayons X ; un détecteur (4) d'image ; une unité de commande (8) de la source (3) et du détecteur (4), le procédé comprenant les étapes consistant à : déterminer (100) au moins une trajectoire de la source (3) et du détecteur (4) en fonction de paramètre(s) de commande, préalablement fixé(s), au moyen de l'unité de commande (8) ; localiser (200) dans l'espace un ou plusieurs objet(s) pouvant être sur la trajectoire ainsi déterminée (2); vérifier (300) la trajectoire déterminée avec la localisation d'un ou plusieurs objet(s) localisé(s) pour détecter, si en fonctionnement, la source et le détecteur décrivant la trajectoire déterminée risque(nt) d'entrer en collision avec un objet localisé.

## Description

### DOMAINE TECHNIQUE GENERAL

L'invention concerne le domaine de l'imagerie médicale à rayons X. Plus particulièrement, elle concerne un procédé de contrôle d'un dispositif d'imagerie médicale à rayons X comprenant une source de rayons X et un détecteur, de préférence reliés par un arceau, pour éviter les collisions entre l'environnement du dispositif d'imagerie et la source et/ou le détecteur.

### ETAT DE LA TECHNIQUE

En imagerie médicale, un dispositif d'imagerie médicale avec un bras en C (plus connu sous le nom d'arceau ou en anglais « *C-arm* ») est utilisé pour examiner un patient.

En effet, un tel dispositif permet d'acquérir des images du patient sans avoir à bouger ce dernier.

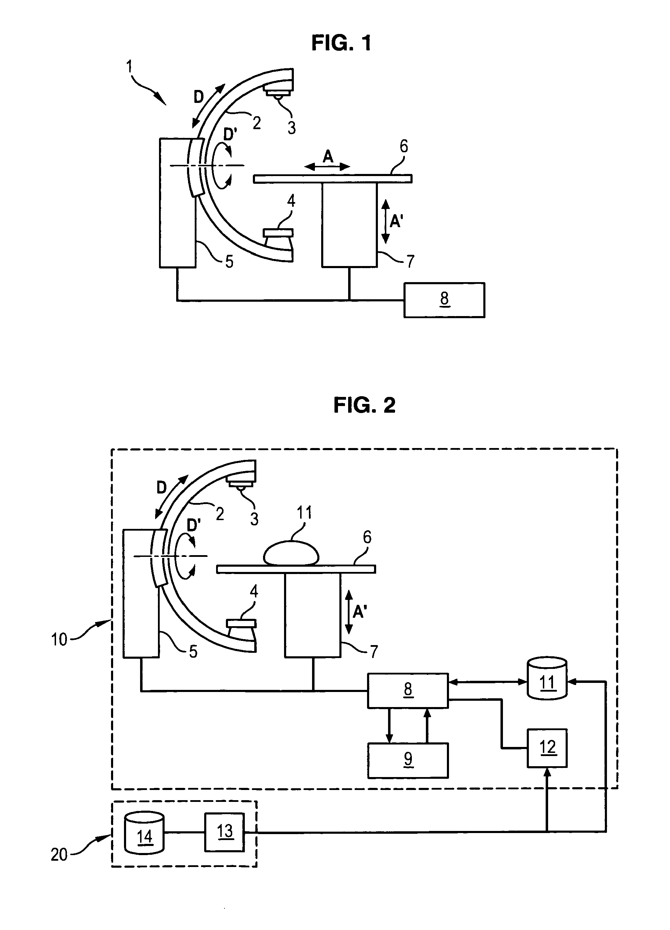

La figure 1 illustre un dispositif 1 d'imagerie médicale à rayons X comprenant un arceau 2 sur lequel sont disposés l'un en face de l'autre une source à rayons X 3 et un détecteur 4. L'arceau 2 est monté sur un support 5.

L'arceau 2 peut être déplacé selon différentes directions D, D' par rapport au support 5.

Le dispositif 1 d'imagerie médicale comprend une table 6 destinée à recevoir un objet à imager, un patient par exemple. La table 6 est disposée sur une base 7 et est déplaçable selon plusieurs directions A, A' par rapport à la base 7.

Au cours de certains types d'acquisitions d'images d'un patient, dites acquisitions rotationnelles ou acquisitions 3D, l'arceau est mis en rotation, au moyen d'une unité de commande 8, autour du patient, la vitesse de rotation de l'arceau peut selon les cas, être élevée.

Ainsi, la table 6 et la base 7 doivent être positionnées correctement pour ne pas que l'arceau 2 entre en collision avec ces derniers et le cas échéant avec le patient et de manière générale avec l'environnement du dispositif.

Pour ce faire, avant de procéder à l'acquisition en tant que telle, un opérateur effectue une rotation dite de test en commandant la rotation de l'arceau à faible vitesse pour vérifier qu'il n'y aura pas de collisions au cours de l'acquisition en tant que telle.

Si la rotation de test montre la présence d'une collision, l'opérateur doit déplacer la table 6 et recommencer la rotation de test.

Par ailleurs, il n'est pas facile pour l'opérateur de savoir quel est le déplacement minimum de la table la table 6 nécessaire pour être sûr d'éviter une collision.

Si l'opérateur déplace trop la table, il évitera certes la collision, mais il encourt le risque que la région d'intérêt qu'il veut imager ne soit plus présente dans l'image.

Une autre difficulté est que, en déplaçant la table 5 pour éviter une collision avec un des éléments de l'arceau, par exemple la source à rayons X 3, l'opérateur peut créer une collision avec un autre élément (par exemple le détecteur 4).

Pour ces raisons, il n'est pas rare qu'en pratique l'opérateur doive effectuer plusieurs rotations de tests (deux, trois ou quatre) jusqu'à trouver une position acceptable pour la table.

Ceci présente l'inconvénient d'allonger la durée de la procédure d'acquisition et est long et fastidieux pour l'opérateur de sorte que bien que présentant des avantages en termes d'acquisition, de tels dispositifs d'imagerie médicale sont finalement peu utilisés.

### PRESENTATION DE L'INVENTION

Un but de l'invention est de pallier les inconvénients précités.

Ainsi, selon un premier aspect, l'invention concerne un procédé de contrôle d'un dispositif d'imagerie médicale au moyen d'une unité anti-collision d'un dispositif d'imagerie médicale, le dispositif d'imagerie médicale comprenant; une source de rayons X ; un détecteur d'image ; une unité de commande de la source et du détecteur, le procédé comprenant les étapes consistant à : déterminer au moins une trajectoire de la source et du détecteur en fonction de paramètre(s) de commande, préalablement fixé(s), au moyen de l'unité de commande ; localiser dans l'espace un ou plusieurs objet(s) pouvant être sur la trajectoire ainsi déterminée ; vérifier la trajectoire déterminée avec la localisation d'un ou plusieurs objet(s) localisé(s) pour détecter, si en fonctionnement, la source et le détecteur décrivant la trajectoire déterminée risque(nt) d'entrer en collision avec un objet localisé.

D'autres aspects du procédé sont les suivants :
- si la vérification de la collision est positive, une étape consistant à déterminer des paramètres de positionnement d'un ou plusieurs objet(s) localisé(s) tels que la source et le détecteur décrivant la trajectoire déterminée n'entrent pas en collision avec l'objet ;
- si la vérification de la collision est positive, une étape consistant à indiquer qu'un ou plusieurs objet(s) se trouvent sur la trajectoire, une étape consistant à déplacer le ou les objet(s) indiqué(s) et à répéter les étapes de localisation et vérification avec la position du ou des objet(s) déplacé(s) ;
- si la vérification de la collision est positive une étape consistant à indiquer que les paramètres de commande sont incorrects ;
- si la vérification de la collision est négative, une étape consistant à indiquer que les paramètres de commande sont corrects ;
- il comprend les étapes consistant à commander la source et le détecteur selon la trajectoire déterminée à une vitesse différente de la vitesse utilisée lorsque le dispositif d'imagerie médicale acquiert des images ;
- la trajectoire de l'arceau est déterminée à partir d'un modèle de trajectoire cylindrique.

Selon un second aspect, l'invention concerne un dispositif d'imagerie médicale comprenant une unité anti-collision comprenant des moyens pour la mise en oeuvre d'un procédé selon le premier aspect de l'invention.

Grâce au procédé et au dispositif selon les premier et second aspects de l'invention un opérateur peut connaître à l'avance si la position des objets situés dans l'environnement proche de l'arceau vont provoquer des collisions sans avoir à pratiquer un test de rotation.

Ainsi, l'invention permet de réduire la durée des procédures d'acquisition au moyen d'un dispositif d'imagerie médicale comprenant un arceau ce qui le rend plus attractif au-delà des avantages qu'il peut procurer en termes de performances.

Enfin selon un troisième et dernier aspect, l'invention concerne un programme d'ordinateur comprenant des instructions machine pour la mise en oeuvre d'un procédé selon le premier aspect de l'invention.

### PRESENTATION DES FIGURES

D'autres caractéristiques et avantages de l'invention ressortiront encore de la description qui suit laquelle est purement illustrative et non limitative et doit être lue en regard des dessins annexés sur lesquels outre la figure 1 brièvement discutée :
- la figure 2 illustre un dispositif d'imagerie médicale conforme à l'invention
- la figure 3 illustre trois trajectoires tridimensionnelles possibles pour l'arceau du dispositif d'imagerie médicale conforme à l'invention ;
- la figure 4 illustre une vue bidimensionnelle d'une table destinée à supporter un objet à imager au moyen d'un dispositif d'imagerie médicale conforme à l'invention ;
- la figure 5 illustre schématiquement des étapes du procédé conforme à l'invention.

### DESCRIPTION DETAILLEE DE L'INVENTION

### Système d'imagerie médicale

La figure 2 illustre un dispositif 10 d'imagerie médicale comprenant, outre les éléments de celui illustré sur la figure 1, une unité 9 anti-collision configurée pour mettre en oeuvre un procédé de contrôler du dispositif 10 d'imagerie médicale et en particulier de déterminer une trajectoire de l'arceau 2 et déterminer la position d'au moins la table 6 par rapport à la trajectoire ainsi déterminée.

L'unité anti-collision 9 est connectée à l'unité de commande 8 du dispositif 10 d'imagerie médicale. La connexion peut être filaire ou sans fil. L'unité anti-collision 9 peut être est par exemple un/des ordinateur(s), un/des processeur(s), un/des microcontrôleur(s), un/des micro-ordinateur(s), un/des automate(s) programmable(s), un/des circuit(s) intégré(s) spécifique(s) d'application, d'autres circuits programmables, ou d'autres dispositifs qui incluent un ordinateur tel qu'une station de travail.

Le détecteur 4 peut être un capteur d'image à semi-conducteurs comprenant, par exemple, du phosphore d'iodure de césium (scintillateur) sur une matrice de transistor/photodiode en silicium amorphe. D'autres détecteurs adéquats sont : un capteur CCD, un détecteur numérique direct qui convertit directement les rayons X en signaux numériques. Le détecteur 4 illustré sur la figure 2 est plan et définit une surface plane d'image, d'autres géométries peuvent bien entendu convenir.

Comme déjà mentionné, l'unité de commande 8 permet de commander l'acquisition notamment en fixant plusieurs paramètres tels que la dose de radiation à émettre par la source 3 à rayons X et le positionnement de la source 3 et du détecteur 4. Elle est de préférence connectée au support 5 de l'arceau 2 par connexion filaire ou sans fil.

L'unité de commande 8 peut comprendre un dispositif de lecture (non représenté) par exemple un lecteur de disquettes, un lecteur de CD-ROM, DVD-ROM, ou des ports de connexion pour lire les instructions du procédé de traitement d'un support d'instructions (non montré), comme une disquette, un CD-ROM, DVD-ROM, ou clé USB ou de manière plus générale par tout support de mémoire amovible ou encore via une connexion réseau.

Une unité de stockage 11 est en outre prévue pour l'enregistrement des paramètres d'acquisition. Il est possible de prévoir que l'unité de stockage 11 est située à l'intérieur de l'unité de commande 6 ou à l'extérieur.

L'unité de stockage 11 peut être formée par un disque dur ou SSD, ou tout autre moyen de stockage amovible et réinscriptible (clés USB, cartes mémoires etc.). L'unité de stockage 11 peut être une mémoire ROM/RAM de l'unité de commande 8, une clé USB, une carte mémoire, une mémoire d'un serveur central.

Le dispositif d'imagerie comprend en outre une unité d'affichage 12 connectée à l'unité de commande 8 pour l'affichage d'images acquises par le dispositif d'imagerie et/ou d'informations sur les paramètres de commande de l'acquisition et/ou sur les informations provenant de l'unité anti-collision 9.

L'unité d'affichage 12 peut être par exemple un écran d'ordinateur, un moniteur, un écran plat, un écran plasma ou tout autre type de dispositif d'affichage de type connu.

Le dispositif d'imagerie médicale 100 est couplé à un système de traitement 200. Le système de traitement 20 comprend une unité de calcul 13 et unité de stockage 14.

Le système de traitement 20 reçoit des images acquises et stockées dans l'unité de stockage 11 du système d'imagerie médicale 10 à partir desquelles il effectue un certain nombre de traitement, par exemple une reconstruction d'une image 3D à partir d'images 2D.

La transmission des données de l'unité de stockage 11 du dispositif d'imagerie médicale 10 vers l'unité de calcul 13 du système de traitement 20 peut être faite à travers un réseau informatique interne ou externe ou à l'aide de tout support mémoire physique adéquat tel que disquettes, CD-ROM, DVD-ROM, disque dure externe, clé USB, carte SD, etc.

L'unité de calcul 13 est par exemple un/des ordinateur(s), un/des processeur(s), un/des microcontrôleur(s), un/des micro-ordinateur(s), un/des automate(s) programmable(s), un/des circuit(s) intégré(s) spécifique(s) d'application, d'autres circuits programmables, ou d'autres dispositifs qui incluent un ordinateur tel qu'une station de travail.

En variante, le calculateur 13 peut comprendre un dispositif de lecture (non représenté) par exemple un lecteur de disquettes, un lecteur de CD-ROM ou DVD-ROM, ou des ports de connexion pour lire les instructions du procédé de traitement d'un support d'instructions (non montré), comme une disquette, un CD-ROM, un DVD-ROM ou une clé USB ou de manière plus générale par tout support de mémoire amovible ou encore via une connexion réseau.

En outre, le système de traitement 20 comprend une unité de stockage 14 pour le stockage des données générées par l'unité de calcul 9.

L'unité de calcul 13 peut être connectée à l'unité d'affichage 12 (comme sur la figure 2) ou bien à une autre unité d'affichage (non représentée).

En outre l'unité de traitement 200 peut être incluse dans le dispositif 10 d'imagerie médicale, leurs unités de stockage 11, 14 respectives dans ce là sont fusionnées.

### Procédé de contrôle

Le procédé de contrôle du dispositif 10 d'imagerie médicale permet de vérifier, sans avoir à commander la rotation de la source et du détecteur qu'il n'y aura pas de collisions entre au moins la table 6 et le détecteur 4 et/ou la table 6 et la source 3 de rayons X.

Comme mentionné ci-dessus, la source 3 et le détecteur 4 peuvent être reliés par un arceau 2. Dans ce cas c'est la trajectoire de l'arceau 2 qui est considérée.

Le procédé de contrôle du dispositif d'imagerie médicale comprend les étapes consistant à :
- déterminer 100 une trajectoire de l'arceau en fonction de paramètres de commande de l'arceau, préalablement fixés, au moyen de l'unité de commande 8 ;
- localiser 200 dans l'espace un ou plusieurs objet(s) pouvant être sur la trajectoire de l'arceau 2 ;
- vérifier 300 la trajectoire déterminée avec la localisation d'un ou plusieurs objet(s) localisé(s) pour détecter, si en fonctionnement, la source et le détecteur décrivant la trajectoire déterminée risque(nt) d'entrer en collision avec un objet localisé.

On explicite ci-dessous les différentes étapes du procédé.

### Détermination de la trajectoire 100

La trajectoire est de préférence déterminée en fonction des paramètres du dispositif nécessaires à l'acquisition d'images de l'unité de commande 8 : position de la source 3, du détecteur 4, le cas échéant de l'arceau 2, dans l'espace.

On note que la position de la source et du détecteur par rapport à la table 6 est conditionnée par la position d'un objet 11 à imager, un patient en pratique, disposé sur la table 6.

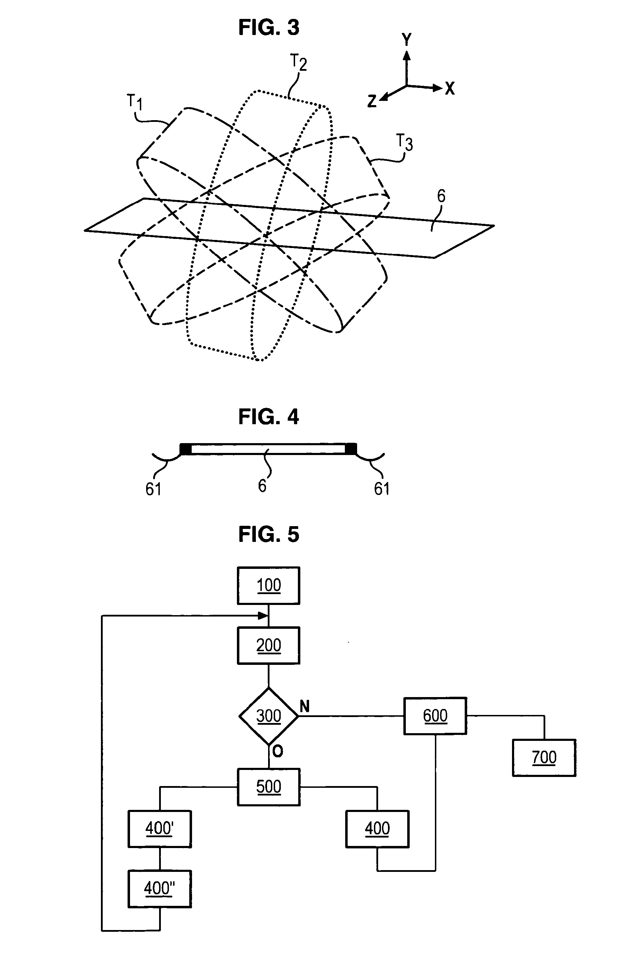

La trajectoire la source et du détecteur la plus simple et la plus utilisée est lorsqu'ils sont reliés par un arceau 2 et est telle que la source de rayons X 3 se déplace suivant un arc de cercle comme cela est illustré sur la figure 3 où trois trajectoires T1, T2, T3 de l'arceau 2 sont représentées.

On peut également envisager d'autres trajectoires plus complexes telles que : source de rayons X 3 se déplaçant suivant un premier arc de cercle dans un premier plan, suivi d'un deuxième arc cercle dans un deuxième plan, ou bien source de rayons X 3 se déplaçant suivant une trajectoire non circulaire (telle que trajectoire elliptique, trajectoire non planaire, etc).

### Localisation 200

On précise que l'on entend par objet à localiser tout objet se situant à proximité du dispositif ou bien dont il est évident qu'il peut être sur la trajectoire de l'arceau 2.

Il peut s'agir de la table 6 destinée à supporter un patient, de la base 7 supportant le patient, du patient lui-même ou bien encore : de moniteurs utilisés pour assister un praticien, un chariot destiné à supporter une interface utilisateur qui permet de commander le dispositif d'imagerie, des accessoires permettant de maintenir la patient sur la table, tels que repose-bras ou repose-tête, et enfin divers dispositifs tels que tables pour poser des instruments médicaux, chariots d'anesthésie, etc.

On a représenté sur la figure 5 la table 6 comportant des supports 61 pour par exemple les bras d'un patient.

Pour localiser 200 dans l'espace au moins un des objets ci-dessus mentionnés on dispose de plusieurs moyens connus par l'homme de l'art :
1) capteurs électro-mécaniques : La localisation de la table 6 est réalisée, de manière connue, par des capteurs électro-mécaniques (encodeurs, potentiométres, etc).
2) capteur permettant de détecter la présence ou l'absence d'un accessoire tel que repose-bras ou repose-tête.
3) capteurs de position de type électromagnétique ou optique, disposés sur chacun des éléments tels que moniteurs, chariots d'anesthésie, etc.
4) capteurs capacitifs, qui permettent de mesurer la distance entre le capteur et l'objet le plus proche. Ils permettent de détecter tous les matériaux, ils fonctionnent sans contact avec le matériau dont on veut évaluer la proximité avec le capteur et ils sont résistants à l'usure. Ils peuvent être utilisés en particulier pour déterminer la position exacte et l'enveloppe du patient 11 sur la table 6. Dans cet objectif, ces capteurs peuvent être fixés par exemple sur le détecteur 4. Ils peuvent également être utilisés pour déterminer la présence ou l'absence d'objets au voisinage de l'arceau.

### Vérification 300

La vérification 300 consiste à vérifier la trajectoire déterminée avec la localisation d'un ou plusieurs objet(s) localisé(s) pour détecter, si en fonctionnement, la source 3 et le détecteur 4 décrivant la trajectoire déterminée risque(nt) d'entrer en collision avec un ou plusieurs objet(s) localisé(s).

De manière plus précise, au cours de cette étape vérification, le ou les objet(s) localisé(s) vont être mis en correspondance avec la trajectoire déterminée décrite par la source 3 et le détecteur 4 pour vérifier où le ou les objet(s) localisé(s) se trouve(nt) par rapport à la trajectoire déterminée.

En effet, à partir de la trajectoire déterminée et des coordonnées du ou des objet(s) localisé(s), il est possible d'effectuer cette vérification 300.

Dans le cas où la vérification 300 est positive, c'est-à-dire qu'une collision est possible entre le ou les objet(s) localisé(s) et la source 3 de rayons X et/ou le détecteur 4, le procédé comprend une étape consistant à déterminer 400 des paramètres de positionnement d'un ou plusieurs objet localisé(s) tels que la source et le détecteur décrivant la trajectoire déterminée n'entrent pas en collision avec l'objet.

En variante, si la vérification de la collision est positive, le procédé peut comprendre une étape consistant à indiquer 400 qu'un ou plusieurs objet(s) se trouve(nt) sur la trajectoire, une étape consistant à déplacer 400" le ou les objet(s) indiqué(s) et à répéter les étapes de localisation 200 et vérification 300 avec la position du ou des objet(s) déplacé(s).

L'étape consistant à déplacer 400" le ou les objet(s) indiqué(s) peut être automatisée.

Dans cette variante, en temps réel, il est donc possible de savoir si l'objet qui est déplacé va provoquer une collision.

On peut envisager des moyens d'alertes visuels ou sonores pour indiquer le statut de l'étape de vérification 300.

On peut en particulier envisager d'afficher 500 sur un dispositif d'affichage un message d'alerte du type « collision » tant que la vérification 300 est positive et afficher 600 un message d'alerte du type « pas de collision » lorsque la vérification 300 est négative.

Dans le cas où la vérification 300 s'avère négative, c'est-à-dire qu'aucune collision n'est possible entre le ou les objet(s) localisé(s) et la source 3 de rayons X et/ou le détecteur 4 le procédé comprend une étape consistant à afficher que les paramètres de commande du dispositif d'imagerie médicale sont corrects.

Enfin, le procédé comprend une étape au cours de laquelle l'arceau est mis en rotation 700 en fonction des paramètres vérifiés.

## Revendications

1. Procédé de contrôle d'un dispositif d'imagerie médicale au moyen d'une unité anti-collision d'un dispositif d'imagerie médicale, le dispositif d'imagerie médicale comprenant; une source (3) de rayons X ; un détecteur (4) d'image ; une unité de commande (8) de la source (3) et du détecteur (4), le procédé comprenant les étapes consistant à :
- déterminer (100) au moins une trajectoire de la source (3) et du détecteur (4) en fonction de paramètre(s) de commande, préalablement fixé(s), au moyen de l'unité de commande (8) ;
- localiser (200) dans l'espace un ou plusieurs objet(s) pouvant être sur la trajectoire ainsi déterminée (2);
- vérifier (300) la trajectoire déterminée avec la localisation d'un ou plusieurs objet(s) localisé(s) pour détecter, si en fonctionnement, la source et le détecteur décrivant la trajectoire déterminée risque(nt) d'entrer en collision avec un objet localisé.

2. Procédé de contrôle selon la revendication 1, comprenant, si la vérification de la collision est positive, une étape consistant à déterminer (400) des paramètres de positionnement d'un ou plusieurs objet(s) localisé(s) tels que la source et le détecteur décrivant la trajectoire déterminée n'entrent pas en collision avec l'objet.

3. Procédé de contrôle selon la revendication 1, comprenant, si la vérification de la collision est positive, une étape consistant à indiquer (400') qu'un ou plusieurs objet(s) se trouvent sur la trajectoire, une étape consistant à déplacer (400") le ou les objet(s) indiqué(s) et à répéter les étapes de localisation et vérification avec la position du ou des objet(s) déplacé(s).

4. Procédé de contrôle selon l'une des revendications 2 à 3, comprenant, si la vérification de la collision est positive une étape consistant à indiquer (500) que les paramètres de commande sont incorrects.

5. Procédé de contrôle selon l'une des revendications 2 à 3, comprenant, si la vérification de la collision est négative, une étape consistant à indiquer (600) que les paramètres de commande sont corrects.

6. Procédé de contrôle selon l'une des revendications 1 à 5, comprenant les étapes consistant à commander (700) la source et le détecteur selon la trajectoire déterminée à une vitesse différente de la vitesse utilisée lorsque le dispositif d'imagerie médicale acquiert des images.

7. Procédé de contrôle selon l'une des revendications 1 à 6 dans lequel la trajectoire de l'arceau est déterminée à partir d'un modèle de trajectoire cylindrique.

8. Dispositif d'imagerie à rayons X comprenant
- une source (3) de rayons X ;
- un détecteur (4) d'image ;
le dispositif comprenant une unité (9) anticollision comprenant des moyens pour mettre en oeuvre un procédé selon l'une des revendications 1 à 7.

9. Dispositif selon l'une des revendications précédentes comprenant un arceau (2) le détecteur (4) et la source (3) de rayons X étant disposés sur des extrémités opposées dudit l'arceau (2).

10. Programme d'ordinateur comprenant des instructions machine pour la mise en oeuvre d'un procédé selon l'une des revendications 1 à 7.
